(19) Europäisches Patentamt
European Patent Office
Office européen des brevets

(11) Publication number: **0 175 402**
**B1**

(12) # EUROPEAN PATENT SPECIFICATION

(45) Date of publication of patent specification: **17.01.90**

(51) Int. Cl.$^5$: **C 07 D 271/10**, G 03 G 5/06

(21) Application number: **85201346.5**

(22) Date of filing: **23.08.85**

(54) **Charge-transporting compounds and photoconductive elements provided with such charge-transporting compounds.**

(30) Priority: **05.09.84 NL 8402705**

(43) Date of publication of application:
**26.03.86 Bulletin 86/13**

(45) Publication of the grant of the patent:
**17.01.90 Bulletin 90/03**

(84) Designated Contracting States:
**CH DE FR GB IT LI NL SE**

(56) References cited:
**EP-A-0 085 447**
**FR-A-1 176 457**

(73) Proprietor: **Océ-Nederland B.V.**
**St. Urbanusweg 43**
**NL-5914 CC Venlo (NL)**

(72) Inventor: **Everhardus, Roelof Hendrik**
**Diepstraat 9**
**NL-5943 BA Lomm (NL)**
Inventor: **Bouts, Wilhelmus Joannes**
**Gouverneur van Hövelllaan 27**
**NL-5953 CT Reuver (NL)**
Inventor: **Brands, Johanbes Jozef Maria**
**Trip 27**
**NL-5944 CS Arcen (NL)**

(74) Representative: **Hanneman, Henri W.A.M. et al**
**Océ-Nederland B.V. Patents and Information**
**Postbus 101**
**NL-5900 MA Venlo (NL)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to charge-transporting compounds and to a photoconductive element comprising a support and one or more layers containing at least a charge-generating compound and a charge-transporting compound which is homogeneously distributed in a binder.

Charge-transporting compounds and their use in photoconductive elements are generally known for example from FR—A—1176457. The charge-transporting compound may be present together with the charge-generating compound in one layer on an electrically conductive support or alternatively be used in a two-layer system in which the charge-generating compound is provided in a charge-generating layer on the support and the charge-transporting compound is provided in a charge-transporting top layer. In this latter type of photoconductive elements, the charge-transporting layer must satisfy stringent requirements in respect of the following properties:

— mechanical strength
— uniformity and film forming
— transparency to visible light
— adhesion to the charge-generating layer
— barrier for surface charge in the dark
— charging value
— hole or electron-transporting capacity
— absence of an injection barrier on the contact surface with the charge-generating layer
— cleanability
— minimal residual charge after exposure.

Most of the known charge-transporting compounds and the charge-transporting layers formed therewith, however, have shortcomings in respect of one or more of the above properties. Charge-transporting layers which are satisfactory in respect of all the above properties to a high degree are those in which the charge-transporting compound is an azine selected from the group described in European Patent Application 0 085 447. Although excellent photoconductive elements can be made with the said azines, there is a need for further improvement.

The object of the invention is to satisfy this need by providing new charge-transporting compounds of the general formula:

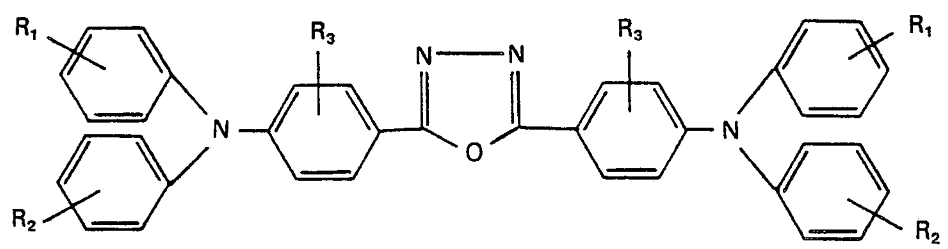

wherein $R_1$ up to $R_3$ inclusive represent a hydrogen atom or an alkyl group containing 1 up to 4 inclusive carbon atoms. The compounds of the general formula in which $R_1$ up to $R_3$ represent a hydrogen atom or a methyl group are preferred. The best results are obtained with 2,5-bis-(p-di-p-tolylamino-phenyl)-1,3,4-oxadiazole, i.e. the compound of the general formula wherein $R_1$ and $R_2$ represent a methyl group in the para position and $R_3$ represents a hydrogen atom. It has been found that 2,5-bis(p-di-p-tolylamino-phenyl)-1,3,4-oxadiazole is soluble more satisfactorily in various solvents and film-forming polymers, thus reducing the risk of crystallization phenomena during the layer formation, and hence providing greater maneouvrability in processing into layers. Like the azines described in European patent application 0 085 447, the latter oxadiazole provides a photoconductive element which satisfies all the above properties, and particularly minimal residual charge, good cleanability and substantial absence of an injection barrier on the contact surface with the charge-generating layer.

The above properties are particularly apparent in photoconductive elements comprising a charge-transporting layer with a charge-transporting compound according to the invention disposed on a thin charge-generating layer. Copies of high quality can be obtained on these materials, even in the event of partial charging. In these two-layer systems, the compounds according to the invention also have the advantage that, as contrasted with the yellow-coloured azines, they form substantially colourless layers so that more light can penetrate into the underlying charge-generating layer and thus it is possible to achieve a gain in photosensitivity ranging up to a considerable gain depending on the light source used. It has also been found that the resistance of these elements to repeated use in the same electrophotographic processes is somewhat higher than that of comparable elements having an azine according to European Patent Application 0 085 447.

The amount of charge-transporting agent in the charge-transporting layer of the photoconductive element according to the invention may vary within wide limits. It is generally between 15% by weight and

70% by weight based on the total quantity of solid. It is preferably between 20% by weight and 40% by weight.

The insulating binder used for the charge-transporting layer of the element according to the invention may be any material suitable for that purpose, e.g. polystyrenes, silicone resins, polyesters of acrylic and methacrylic acid, vinyl polymers and vinyl copolymers. Particularly good results are obtained with polycarbonates, because of their high transparency, mechanical strength and good adhesion to the photoconductive layer.

Any support known for that purpose may be used in the photoconductive element according to the invention.

Such supports may of themselves be conductive, such as e.g. supports of aluminium, steel or nickel, or they may have been made conductive, such as e.g. paper or plastics supports to which a thin conductive layer, e.g. of aluminium or nickel, has been applied. For use in indirect electrophotography, for which the present element is extremely suitable because of its special properties, the support will generally be endless in form, e.g. a drum or a flexible web of paper or plastics, the ends of which are interconnected.

The radiation-sensitive charge-generating compound to be used in the charge-generating layer of the element according to the invention may be inorganic or organic. Examples of the former are selenium or amorphous silicon. However, organic compounds are preferably used for the charge-generating compound, more particularly the radiation-sensitive bis-azo compounds described in US Patent 4 052 210. The thickness of the photoconductive layer is preferably between about 0.2 and 2 μm.

In a specific embodiment, the charge-transporting layer of the electrophotographic element according to the invention comprises one or more activators.

Particularly if it is desired to charge the element only partially, say to 30—70% of its maximal chargeability ($ASV_{max}$), in order to obtain a higher permanence, the use of an activator is usually desirable to improve the discharge characteristics.

In principal, any activators known for that purpose may be used.

Examples of usable activators are trinitrofluorenone, the dibenzothiophene oxides referred to in US Patent 3 905 814 and the N-(fluorene-9-ylidene)-anilines referred to in US Patent 3 935 009. Particularly good results were obtained with the activators terephtalaldimalononitrile (hereinafter referred to as TDM) and 1,3,7-trinitrodibenzothiophene-5,5-dioxide.

As contrasted with many activators used hitherto, TDM is also absolutely non-mutagenic. For most activators the amount required is generally between 1 and 15% by weight based on the charge-transporting agent. If TDM is used in combination with a charge-transporting agent according to the invention, the required results can already be obtained with quantities between 0.5 and 3% by weight.

The electrophotographic element according to the invention can be made according to any of the processes described in the patent specifications referred to thereinbefore. Both the preparation of the charge-generating layer and of the charge-transporting layer are described in detail therein. The charge-transporting compounds according to the invention can be prepared by the reaction of hydrazine with p-aminobenzoic acid, which may or may not be alkyl-substituted, in oleum and then reacting the resulting 2,5 bis-(p-aminophenyl)-1,3,4-oxadiazole with iodobenzene or alkyl iodobenzene in the presence of copper powder and potassium carbonate.

The latent image formed in the conventional way on the charge-transporting layer can be made visible both by means of a two-component and a one-component developer. In the former case the developer consists of relatively coarse carrier particles, usually of iron, and very finely divided toner particles which obtain the required polarity by contact with the carrier particles. In the latter case the developer consists basically of finely divided toner particles only, which may be conductive (resistivity less than $10^{10}$ Ohm.m) or insulating (resistivity greater than $10^{10}$ Ohm.m).

The photoconductive element according to the invention has been found very suitable for developing with a one-component developer, and this has a number of advantages. When an insulating one-component developer is used it is desirable to provide the photoconductive element according to the invention with a function layer in the form of a screen which produces a screen pattern in the image parts. Specific types of layers and the place and method of application are known to those versed in art. They are described, inter alia, in the book "Xerography and related processes" by Dessauer and Clark, 1965, pp. 112—117.

When a conductive one-component developer is used, it is desirable to provide the photoconductive element with a screen structure which on imagewise exposure of the charged element causes a charge screen in the image background. A photoconductive element provided with a screen of this kind is described in the Dutch patent application 84 00 922, which is not a prior publication.

The invention will be explained in detail with reference to the following examples.

## Example 1

a) Preparation of 2,5-bis-(p-aminophenyl)-1,3,4-oxadiazole

60 g (0.46 mol) of hydrazine sulphate were dissolved in 700 ml of oleum with 20% $SO_3$.

The following was added at room temperature:

137 g (1.0 mol) p-aminobenzoic acid.

The mixture was stirred for 2½ hours at a temperature of 70—75°C, then poured on to 10 litres of ice and

neutralized with a concentrated sodium hydroxide solution. The precipitate was filtered off, washed with water and again filtered off. The yield was 91 g impure product.

This product was recrystallized in ethanol. After partial evaporation of the liquid, 73.5 g of pure 2,5-bis-(p-aminophenyl)-1,3,4-oxadiazole were obtained.

b) Preparation of 2,5-bis-(p-di-p-tolylamino-phenyl)-1,3,4-oxadiazole.

The following products were added to one another:

40.8 g (0.16 mol) of 2,5-bis-(p-aminophenyl)-1,3,4-oxadiazole
157,8 g (0.72 mol) of p-iodotoluene
6,0 g copper powder
152.0 g anhydrous potassium carbonate
400 ml nitrobenzene.

The mixture was kept at 210°C with reflux for 7.5 hours with continuous removal of water. The nitrobenzene was then removed by steam distillation and the residue extracted with toluene and reduced by evaporation.

The solid obtained by evaporation (78 g) was recrystallized with 3 litres of ethyl acetate. After evaporation of the liquid to 500 ml, 54 g of pure 2,5-bis-(p-di-p-tolylamino-phenyl)-1,3,4-oxadiazole were filtered off.

Example 2

A charge-generating layer containing the blue bis-azo dye 3,3'-dimethoxy-4,4'-bis(2''-hydroxy-3''-anilinecarbonyl naphthylazo-) biphenyl in molecularly divided form in a binder was prepared as follows: A solution of 1.2 g of cellulose acetate butyrate in 60 ml of acetone was mixed with a solution of 1 g of 2-hydroxy-N-phenyl-3-naphtalene carboxamide in 13 ml of N,N-dimethylformamide. A solution of 0.5 g of 4,4'-biadiazonium boron tetrafluoride salt of 3,3'-dimethoxybiphenyl in 7 ml of N,N-dimethyl formamide was added to this mixture. The resulting mixture was kept in the dark for 10 minutes and then applied to a conductive support (polyester film with a vapour-coated aluminium layer) by dip coating at 25 to 30°C and 30 to 40% relative humidity. After drying, coupling to the above-mentioned bis-azo compound took place in situ by treatment with ammonia. The thickness of the charge-generating layer was 0.3 µm.

A charge-transporting layer was applied to this charge-generating layer by dip coating using the following solution:

25 ml of 10% polycarbonate (Lexan-141 made by Messrs. General Electric) in 1,2-dichloroethane (i.e.: 2.5 g polycarbonate in 25 ml binder solution), 1.5 g of 2,5-bis-(p-di-p-tolylamino-phenyl)-1,3,4-oxadiazole and 8 ml of tetrahydrofuran with 0.03 g of the activator terephthalaldimalonitrile dissolved therein.

After drying for 15 minutes to the air, the resulting double layer was dried at 105°C in vacuo for 30 minutes.

Photocopies were made in an indirect photocopying machine using the resulting multi-layer electrophotographic element. The following were examined: layer thickness, layer adhesion strength, charging, dark discharge, photo-sensitivity, surface charge density, residual voltage, memory effect, image quality of the copy obtained after the image developed on the charge-transporting layer by means of an electrically conductive one-component developer had been transferred to ordinary paper, followed by fixing by heat and pressure, and permanence. The results obtained with the electrophotographic element according to this example, and the results obtained with the elements according to Examples 3—9, are summarised after Example 9.

Example 3 (comparative example)

Same as Example 2 except that the azine of 4-(di-4'-tolyl)aminobenzaldehyde was used as charge-transporting compound instead of the oxadiazole mentioned.

Example 4

A charge-generating layer was prepared which contained the purple bis-azo dye 4,4'-bis(2''-hydroxy-3''-isopropylaminocarbonylnaphthylazo-)-stilbene as the charge-generating compound, in the form of small pigment particles (about 0.2 µm) distributed in a binder. For this purpose, 1 g of the said bis-azo dye was dispersed in a solution of 1 g of cellulose acetate butyrate in 50 ml of 1,2-dichloroethane and 10 ml tetrahydrofuran by grinding for 24 hours in a ball mill.

This preparation was applied by dip coating to a conductive support (polyester film with a vapour-coated aluminium layer). After drying, the thickness of this charge-generating layer was 1.0 µm. A charge-transporting layer was applied to this charge-generating layer by dip coating with 2,5-bis-(p-di-p-tolylaminophenyl)-1,3,4-oxadiazole, as described in Example 2.

Example 5 (comparative example)

Same as Example 4 except that the azine of Example 3 was used as the charge-transporting compound instead of the said oxadiazole.

Example 6

A charge-generating layer was prepared which contained the purple bis-azo dye 4,4'-bis(2''-hydroxy-3''-isopropylamino-carbonylnaphthylazo-)-stilbene and the red polycondensation product of p-phenylene-

bis-acetonitrile and 2,5 dimethoxy terephthalaldehyde (described in East German Patent 75233) as the charge generating compounds, in the form of small pigment particles (about 0.2 μm) distributed in a binder.

For this purpose, 0.75 g of the above bis-azo dye and 0.75 g of the above polymeric dye were finely dispersed, after the addition of 15 ml of tetrahydrofuran, in a solution of 1.5 g of polyvinyl chloride-polyvinyl acetate copolymer (VMCH made by N.V. Contivema) in 60 ml of 1,2-dichloroethane, by grinding for 72 hours in a ball mill.

This preparation was applied to a conductive support (polyester film with a vapour-coated aluminium layer) by dip coating. The thickness of the charge-generating layer after drying was 0.7 μm. A charge-transporting layer was applied to the charge-generating layer by dip coating as in Example 2 using the oxidiazole according to Example 2.

Example 7 (comparative example)

Same as Example 6 except the azine according to Example 3 was used in the charge-transporting layer instead of the said oxadiazole.

Example 8

A charge-generating layer was prepared with the dark-red pigment N,N'-dibenzyl-perylene-3,4:9,10-tetracarboxylic acid diimide as charge-generating compound. For this purpose, a thin (0.2 μm) layer of the said perylene derivative was applied by vapour-coating to a conductive support (polyester film with a vapour-coated aluminium layer) at a pressure of $10^{-15}$ to $10^{-6}$ torr. A charge-transporting layer was applied to this charge-generating layer as in Example 2 using the oxadiazole according to Example 2.

Example 9 (comparative example)

Same as Example 8 except that the azine according to Example 3 was used in the charge-transporting layer instead of the said oxadiazole.

Results of Examples 2—9

In Examples 3, 5, 7 and 9, after the charge-transporting azine has been dissolved hot the preparation has to be processed rapidly to avoid crystallizing out therein or on the layer. In Examples 2, 4, 6 and 8, the charge-transporting oxadiazole remains completely in solution at room temperature. The charge-transporting layers from the examples were about 4 μm thick. The adhesion strength of the layers was excellent. The memory effect was in every case totally absent on partial charging.

The copy quality using a one-component developer of the layers according to Examples 2, 4, 6 and 8 and those according to their corresponding comparative examples was excellent both with complete and partial charging.

All the layers were easily cleaned using a one-component developer.

The photoelectric results at complete charging are summarised in Table 1, those for partial charging are given in Table 2.

TABLE 1

| | Maximum charging | | | | | |
|---|---|---|---|---|---|---|
| Example | ASV (volt) | D.O.—1 (%) | D.O.—5 % | sigma (mC/m²) | L—20 (mJ/m²) | Residual (%) |
| 2 | 410 | 9 | 21 | 3.3 | 39 | 5 |
| 3 (comparative example) | 420 | 9 | 20 | 3.3 | 40 | 3 |
| 4 | 628 | 7 | 17 | 3.5 | 30 | 7 |
| 5 (comparative example) | 657 | 7 | 15 | 3.1 | 34 | 5 |
| 6 | 415 | 11 | 24 | 2.4 | 18 | 4 |
| 7 (comparative example) | 479 | 14 | 30 | 2.1 | 20 | 3 |
| 8 | 495 | 4 | 12 | 3.7 | 22 | 2 |
| 9 (comparative example) | 423 | 12 | 31 | 2.8 | 23 | 1 |

TABLE 2

| Example | Partial charging | | | | | |
|---|---|---|---|---|---|---|
| | ASV (volt) | D.O.—1 (%) | D.O.—5 % | sigma ($mC/m^2$) | L—20 ($mJ/m^2$) | Residual (%) |
| 2 | 197 | 2 | 8 | 1.8 | 40 | 9 |
| 3 (comparative example) | 198 | 2 | 8 | 1.7 | 40 | 5 |
| 4 | 222 | 4 | 11 | 1.4 | 27 | 8 |
| 5 (comparative example) | 219 | 5 | 15 | 1.0 | 28 | 5 |
| 6 | 229 | 7 | 18 | 1.4 | 17 | 6 |
| 7 (comparative example) | 205 | 10 | 25 | 1.1 | 18 | 5 |
| 8 | 201 | 1 | 2 | 1.6 | 13 | 2 |
| 9 (comparative example) | 200 | 2 | 5 | 1.6 | 15 | 1 |

ASV: surface potential in volts after charging.
D.O.—1 and D.O.—5: dark discharge in 1 and 5 seconds respectively after charging as percent of ASV.
Sigma: surface charge density in $mC/m^2$, measured after 1 second dark discharge.
L—20: amount of light in $mJ/m^2$ to discharge the layer using a flash (BRAUN flash type F900) to 20% of ASV.
Residual: Percentage of ASV remaining after exposure with 100 $mJ/m^2$ (BRAUN flash type F900).

Example 10

To illustrate the high transparency to short-wave light of the charge-transporting layer containing 2,5-bis-(p-di-p-tolylamino-phenyl)-1,2,3,4-oxadiazole and hence the greater photo-sensitivity and colour reproduction of the electrophotographic element of Example 8 compared with that of Example 9, which contained the yellow azine of 4-(di-4'-tolyl) aminobenzaldehyde, the photoconductive elements from both examples were exposed through different narrow band filters after charging. The photoelectric results using filters with transmission maxima of 411—526 nm respectively are given in Table 3.

At higher wavelengths than given in Table 3, the layers of both examples behave identically. The symbols have the same meanings as in Tables 1 and 2.

TABLE 3

| In both cases ASV = 210 Volt, sigma = 1.6 $mc/m^2$, D.O.—1 example 8 = 1%, D.O.—1 example 9 = 2% | | |
|---|---|---|
| Wavelength (nm) | L—20 example 8 ($mJ/m^2$) | L—20 example 9 ($mJ/m^2$) |
| 411 | $>100$ | $\gg100$ |
| 435 | 17 | $>100$ |
| 463 | 8 | $>100$ |
| 476 | 8 | 20 |
| 488 | 8 | 9 |
| 501 | 7 | 7 |
| 526 | 7 | 7 |

**Claims**

1. Charge-transporting compounds of the general formula I

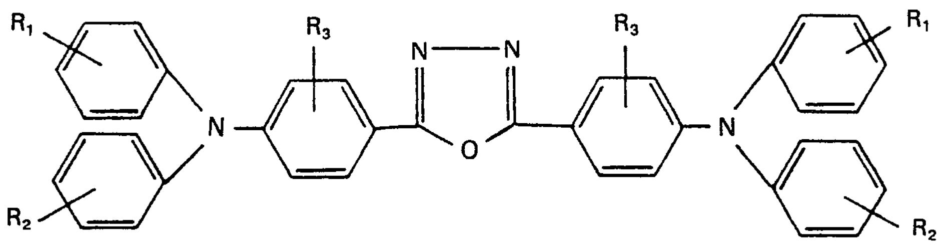

wherein $R_1$ up to $R_3$ inclusive represent a hydrogen atom or an alkyl group containing 1 up to 4 inclusive carbon atoms.

2. Charge-transporting compounds of the general formula I in which $R_1$ up to $R_3$ represents a hydrogen atom or a methyl group.

3. Charge-transporting 2,5-bis-(p-di-p-tolylamino-phenyl)-1,3,4-oxadiazole.

4. Photoconductive element comprising a support and one or more layers together containing at least a charge-generating compound and a charge-transporting compound which is homogeneously distributed in a binder, characterised in that the charge-transporting compound is a compound according to claim 1.

5. Photoconductive element according to claim 4, characterised in that the charge-transporting compound is a compound according to claim 2.

6. Photoconductive element according to claim 4, characterised in that the charge-transporting compound is 2,5-bis(p-di-p-tolylaminophenyl)-1,3,4-oxadiazole.

**Patentansprüche**

1. Ladungsübertragende Verbindungen gemäß der allgemeinen Formel I

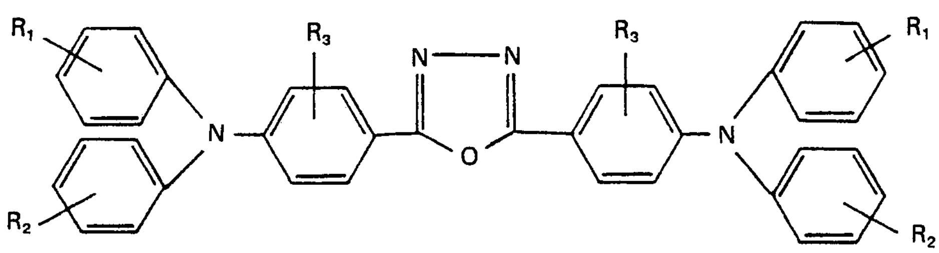

wobei $R_1$ bis einschließlich $R_3$ ein Wasserstoffatom oder eine Alkylgruppe mit 1, 2, 3 oder 4 Kohlenstoffatomen repräsentieren.

2. Ladungsübertragende Verbindungen gemäß der allgemeinen Formel I, bei der $R_1$ bis $R_3$ ein Wasserstoffatom oder eine Methylgruppe repräsentieren.

3. Ladungsübertragendes 2,5-Bis(p-di-p-Tolylamino-Phenyl)-1,3,4-Oxadiazol.

4. Photoleitendes Element mit und einem Träger einem Träger und einer oder mehrerer Schichten, die zusammen wenigstens eine ladungserzeugende Verbindung und eine ladungsübertragende Verbindung die homogen in einem Bindemittel verteilt ist enthalten, dadurch gekennzeichnet, daß die ladungsübertragende Verbindung eine Verbindung gemäß Anspruch 1 ist.

5. Photoleitendes Element nach Anspruch 4, dadurch gekennzeichnet, daß die ladungsübertragende Verbindung eine Verbindung nach Anspruch 2 ist.

6. Photoleitendes Element nach Anspruch 4, dadurch gekennzeichnet, daß die ladungsübertragende Verbindung 2,5-Bis(p-di-p-Tolylamino-Phenyl)-1,3,4-Oxadiazol ist.

**Revendications**

1. Composés transporteurs de charge de formule générale I

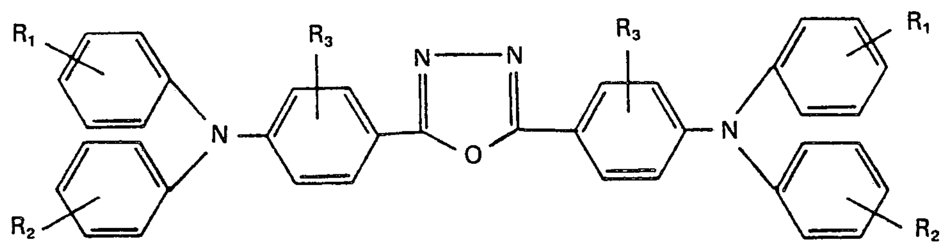

où $R_1$ à $R_3$ représentent un atome d'hydrogène ou un groupe alcoyle renfermant de 1 à 4 atomes de carbone.

2. Composés transporteurs de charge de formule générale I où $R_1$ à $R_3$ représentent un atome d'hydrogène ou un groupe méthyle.

3. 2,5-bis(p-di-p-tolylaminophényl)-1,3,4-oxadiazole transporteur de charge.

4. Elément photoconducteur comprenant un support et une ou plusieurs couches renfermant ensemble au moins un composé générateur de charge et un composé transporteur de charge réparti de façon homogène au sein d'un liant, caractérisé en ce que le composé transporteur de charge est un composé selon la revendication 1.

5. Elément photoconducteur selon la revendication 4, caractérisé en ce que le composé transporteur de charge est un composé selon la revendication 2.

6. Elément photoconducteur selon la revendication 4, caractérisé en ce que le composé transporteur de charge est le 2,5-bis(p-di-p-tolylaminophényl)-1,3,4-oxadiazole.